# EUROPÄISCHE PATENTSCHRIFT

(11) **EP 2 925 456 B1**
(45) Veröffentlichungstag und Bekanntmachung des Hinweises auf die Patenterteilung: **08.03.2017**
(21) Anmeldenummer: 13798581.8
(22) Anmeldetag: 25.11.2013
(51) Int. Cl.: B05B 11/00, B05B 15/02, B65D 83/40

(54) **SCHUTZKAPPE FÜR EINEN SPENDER MIT EINER EINLAGE ZUM REINIGEN DER DÜSE**
PROTECTIVE CAP FOR A DISPENSER HAVING AN INSERT FOR CLEANING THE NOZZLE
COUVERCLE DE PROTECTION POUR UN DISTRIBUTEUR PRÉSENTANT UN INSERT POUR LE NETTOYAGE DE LA BUSE

(30) Priorität: 28.11.2012 DE 102012023215
(43) Veröffentlichungstag der Anmeldung: 07.10.2015
(73) Patentinhaber: MeadWestvaco Calmar GmbH, 58675 Hemer (DE)
(72) Erfinder: THIEDE, Stephan, 58509 Lüdenscheid (DE); GRATZFELD, Thomas, 58636 Islerlohn (DE); WELP, Gisbert, 59846 Sundern (DE)
(74) Vertreter: Henseler, Daniela
(86) Internationale Anmeldenummer: PCT/EP2013/003555
(87) Internationale Veröffentlichungsnummer: WO 2014/082727

(56) Entgegenhaltungen:
- EP-A1- 1 020 135
- FR-A1- 2 750 406
- US-A- 2 249 832
- US-A- 2 737 416

## Beschreibung

Die Erfindung betrifft einen manuell betätigbaren Spender für Medien nach dem Oberbegriff des Anspruchs 1.

Aus DE 44 00 945 A1 ist eine derartige Abgabevorrichtung für fluide Medien bekannt. Ein Fluid-Pumpbehälter besteht aus einem zylinderförmigen Fluidbehälter, auf dem ein eine Dosierpumpe aufweisendes Pumpenteil mittels eines Dichtrings dichtend aufgesetzt ist. Die Dosierpumpe wird durch ein Betätigungselement betätigt, das von einer elastischen Kraft in seiner oberen Ruheposition gehalten wird. Zwischen der oberen Ruheposition und der unteren Betätigungsposition ist das Betätigungselement um einen Betätigungsweg bewegbar. Zur Betätigung weist das Betätigungselement einen tellerförmigen Abschnitt auf. Die Dosierpumpe fördert das Fluid aus dem Fluidbehälter durch einen vertikalen Fluidkanal, der sich axial durch das Betätigungselement nach oben fortsetzt bis zu einer an der Spitze des Betätigungselements angeordneten Austrittsöffnung. Die Spitze des Betätigungselements kann durch eine Verschlusskappe verschlossen werden. Nachteilig ist, dass die Verschlusskappe gebrauchsbedingt durch Fluidreste innenseitig verschmutzen kann.

Aus DE 100 50 982 A1 ist ein Spender, insbesondere ein Zerstäuber für fließfähige Substanzen, besonders Pharmazeutika, bekannt, der eine Pumpe verwendet, die zum Ansaugen ein- oder mehrfach betätigt werden muss. Ihre Austragsdüse ist von einer Schutzkappe überdeckt, die geeignet ist, das Medium aufzufangen und zu speichern, das während der Ansaughübe und vor Beginn der eigentlichen Nutzhübe ausgebracht wird. Die Schutzkappe ist auf dem Spender während der Ansaughübe gesichert. Die Schutzkappe kann die darein gesprühte, gespritzte oder getropfte Medienmenge, z.B. in einem schwammartigen Element, aufnehmen. Zum eigentlichen Betätigungshub wird dann die Schutzkappe abgenommen. In der Schutzkappe ist, da sie, vor allem in ihrem oberen Bereich, einen größeren Abstand von dem Nasenadapter hat, ein Speicherraum ausgebildet. In diesem ist als Speichermittel ein schaum- oder schwammartiges Element vorgesehen, das den Nasenadapter ringförmig umgebend angeordnet ist. Der Speicherraum, der das bei Ansaughüben abgegebene Medium aufnimmt, kann nach außen entlüftet werden. Die Schutzkappe wird hier als Zwischenspeicher für flüssiges Medium genutzt, um die Dosiergenauigkeit zu verbessern. Der eigentliche Gebrauchszweck der Schutzkappe, die Austragöffnung abzudecken und zu schützen, geht hier verloren, da die Schutzkappe zu einem Sprühschild wird.

Aus WO 1993/024164 A1 ist ein Spender mit einer Schutzkappe bekannt. In die Schutzkappe ist eine weiche, porige Einlage eingelegt.

Aus DE 41 37 799 A1 ist ein Spender mit einer Schutzkappe bekannt. In die Schutzkappe des Spenders ist eine Torsionsfeder eingespannt. Ein Ventilkörper ist somit gegenüber der Schutzkappe federnd bewegbar, so dass sie sich besonders gut schließend in einen Ventilsitz einarbeitet.

Aufgabe der Erfindung ist es daher, einen manuell betätigbaren Spender für Medien zu schaffen, bei dem die Schutzfunktion der Schutzkappe für die

Austragöffnung verbessert ist.

Diese Aufgabe wird durch die Merkmale des Anspruchs 1 gelöst.

Hierdurch wird ein manuell betätigbarer Spender für Medien geschaffen, dessen eine Schutzkappe beim Aufsetzen auf den Spender ein Abwischen des die Austragsöffnung aufweisenden Austragabschnittsendes bewirkt. Eine weiche, porige Einlage saugt Medienreste auf, die nach einer Benutzung des Spenders an dessen Austragabschnittsende verblieben sind. Die Torsionsfeder sorgt dabei für eine hinreichende Druckkraft, die als eine Putzkraft wirkt. Das zusammen mit der Druckkraft eingeleitete Drehmoment stellt den für Wischbewegungen typischen Vorgang, etwas durch reibendes Darüberstreichen aufzunehmen, nach. Die weiche und porige Einlage wird erfindungsgemäß nicht nur zur Aufnahme von Medium genutzt, sondern auch als Wischgerät. Das Abnehmen von Medienresten kann zu Reinigungszwecken als auch zu Analysezwecken genutzt werden.

Über die Torsionsfeder kann in Kombination mit einer zur Befestigung der Schutzkappe an dem Austragabschnittsende vorgesehenen Schnappverbindung eine Zwangsbewegung zum Abwischen des Austragabschnittsendes beim Aufsetzen der Schutzkappe auf den Spender ausgelöst werden. Diese Zwangsbewegung kann über eine federnde Länge der Torsionsbewegung ausgelöst werden, die über den Übergang der Rastelemente der Schnappverbindung hinausgeht. Die zum Einschnappen der Schnappverbindung notwendige manuelle Druckkraft führt dann zu einem Überschwingen des Übergangs, bei dem die Schutzkappe mit ihrer Einlage gegen das Austragabschnittsende gedrückt und durch das dabei eingeleitete Drehmoment reibend über das Austragabschnittsende streicht.

Medienrückstände, insbesondere flüssige Medienrückstände, vorausgegangener Abgabevorgänge können so von der Austragöffnung abgenommen werden, wenn die Schutzkappe aufgesetzt wird. Eine Abtrocknung der Einlage kann verbessert werden, wenn die Schutzkappe Lüftungsöffnungen aufweist, vorzugsweise in dem Bereich der Schutzkappe, der die Einlage umgibt.

Als weiche, porige Einlage sind beispielsweise geeignet die Funktionsgegenstände Schwamm, Membran, Vlies oder sonstige textile Materialien aus Natur- oder Kunststofffasern.

Weitere Ausgestaltungen der Erfindung sind der nachfolgenden Beschreibung und den Unteransprüchen zu entnehmen.

Die Erfindung wird nachstehend anhand der in den beigefügten Abbildungen dargestellten Ausführungsbeispiele näher erläutert.
Fig. 1 zeigt schematisch eine Seitenansicht eines manuell betätigbaren Spenders gemäß einem ersten Ausführungsbeispiel,
Fig. 2 zeigt schematisch einen Schnitt von Fig. 1,
Fig. 3 zeigt schematisch eine perspektivische Ansicht eines Teilbereichs des Spenders mit aufgesetzter Schutzkappe nach Entfernen eines Abreißringes,
Fig. 4 zeigt schematisch einen Schnitt von Fig. 3,
Fig. 5 zeigt schematisch eine perspektivische Ansicht eines Teilbereichs des Spenders mit aufgedrückter Schutzkappe,
Fig. 6 zeigt schematisch einen Schnitt von Fig. 5,
Fig. 7 und Fig. 8 zeigen schematisch perspektivische Ansichten eines Teilbereichs des Spenders vor und nach dem Entfernen eines Abreißstreifens für einen Erstgebrauch des Spenders,
Fig. 9 zeigt schematisch eine perspektivische Ansicht eines manuell betätigbaren Spenders gemäß einem zweiten Ausführungsbeispiel,
Fig. 10 und Fig. 11 zeigen schematisch einen Schnitt und eine perspektivische, teilweise zerlegte Ansicht eines manuell betätigbaren Spenders gemäß einem dritten Ausführungsbeispiel.

Die Erfindung betrifft einen manuell betätigbaren Spender für Medien, der zum Austragen insbesondere eines flüssigen Mediums, eines Aerosols oder sonstigen fluiden Mediums, gegebenenfalls mit Feststoffanteilen, vorgesehen ist. Bei den Medien handelt es sich beispielsweise um Pharmazeutika.

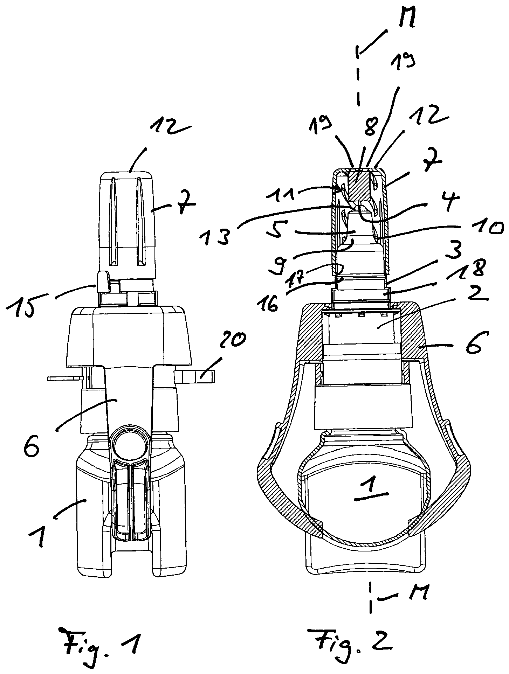

Wie Fig. 1 und Fig. 2 zeigen, umfasst der Spender einen Vorratsbehälter 1 und ein auf dem Vorratsbehälter 1 aufgesetztes Pumpenteil 2. Das Pumpenteil 2 ist gegenüber dem Vorratsbehälter 1 in einer Hubrichtung bewegbar, vorzugsweise zwischen einer oberen Ruheposition und einer unteren Betätigungsposition. Die Hubrichtung ist hier axial ausgerichtet.

Der Aufbau des Pumpenteils 2 kann in bekannter Weise erfolgen und ist beispielsweise beschrieben in DE 10 2008 027 598 A1.

Wie Fig. 2 zeigt, umfasst das Pumpenteil 2 einen stutzenförmigen Austragabschnitt 3 mit einem eine Austragöffnung 4 aufweisenden Austragabschnittsende 5. Dem Pumpenteil 2 ist eine Betätigungseinrichtung 6 zugeordnet, um durch manuelle Betätigung der Betätigungseinrichtung 6 einen Austrag des Mediums zu erzeugen. Zu diesem Zweck ist das Medium in dem Vorratsbehälter 1 bevorratet, aus dem das Medium mittels des Pumpenteils 2 über einen Medienkanal in dem Austragabschnitt 3 über einzelne Pumpenhübe ausgetragen wird. Der stutzenförmige Austragabschnitt 3 kann abhängig vom Anwendungszweck unterschiedlich, gegebenenfalls auch ergonomisch, gestaltet sein, beispielsweise als Nasenadapter oder Tropfadapter. Das Pumpenteil 2 und der Vorratsbehälter 1 sind hier koaxial angeordnet.

Für mindestens das Austragabschnittsende 5 ist eine abdeckende Schutzkappe 7 vorgesehen, die die Austragöffnung 4 und das diese umgebende Austragabschnittsende 5 während des Nichtgebrauchs schützen soll. Verschmutzungen und/oder Beschädigungen des Spenders im Bereich des Austrags des Mediums sollen so vermieden werden.

Die Schutzkappe 7 ist ein mindestens das Austragabschnittsende 5 überdeckender und vorzugsweise buchsenförmiger Deckel, der wiederholt fest aufsitzend anbringbar ist nach einem Gebrauch des Spenders.

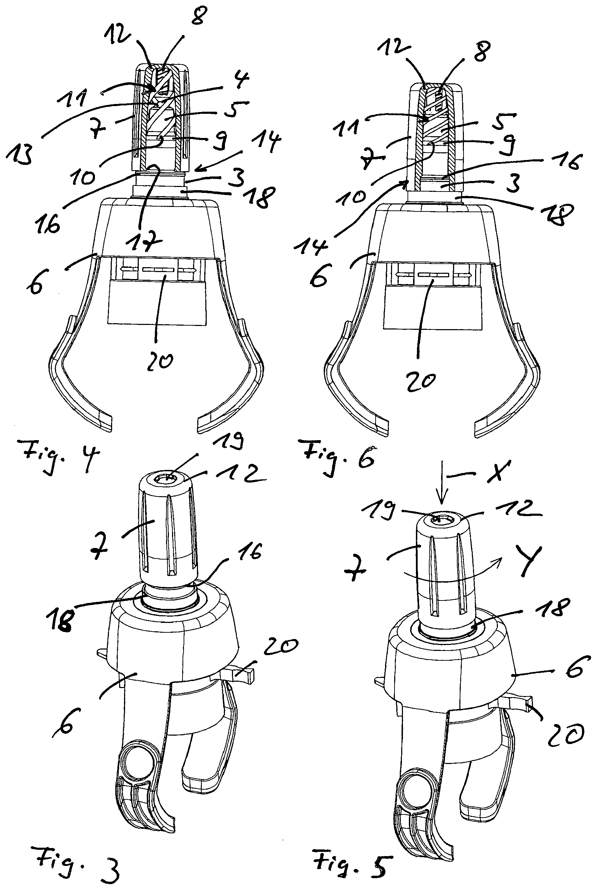

Wie Fig. 2 bis Fig. 5 zeigen, ist in die Schutzkappe 7 eine weiche, porige Einlage 8 eingelegt, die beim Aufsetzen der Schutzkappe 7 von oben an das Austragabschnittsende 5 drückbar ist. Der Austragabschnitt 3 bildet eine Aufstandsfläche 9 für ein freies Ende 10 einer in der Schutzkappe 7 eingespannten Torsionsfeder 11. Die Belastung der Torsionsfeder 11 beim Andrücken der Schutzkappe 7 auf das Austragabschnittsende 5 leitet ein Drehmoment ein, das die Schutzkappe 7 gegenüber dem Austragabschnitt 3 verdreht. Fig. 5 verdeutlicht die Richtungen der Bewegungskomponenten durch die Pfeile X und Y. Die Torsionsfeder 11 wird durch das Zusammendrücken der Enden belastet. Bei Belastung verdreht sich die Schutzkappe 7 um ihre Längsachse. Die Schwenkbewegung in Richtung des Pfeils Y um eine Drehachse erfolgt dann vorzugsweise um eine Mittelachse M (vgl. Fig. 2) des Pumpenteils 2 und/oder des Spenders. Der Drehwinkel ist von der Länge, vorzugsweise einer Windungszahl oder einer Drehstablänge, und Durchmesser der Torsionsfeder und der Festigkeit des verwendeten Federmaterials abhängig. Längere Torsionsfedern 11 ergeben einen größeren Drehwinkel. Ein kleiner Drehwinkel ist im Allgemeinen ausreichend, der beispielsweise 0,5 bis 3 mm betragen kann.

Wie Fig. 2 und Fig. 4 zeigen, ist die Schutzkappe 7 in ihrem Innenraum benachbart zu einer kopfseitigen Schutzkappenwand 12 mit der weichen, porigen Einlage 8 ausgestattet, die vorzugsweise kissenartig ausgebildet ist. Die Einlage 8 besitzt dann vorzugsweise eine axiale Erstreckung in den Innenraum der Schutzkappe 7 hinein, wodurch die Einlage 8 in Axialrichtung ein nachgebendes, Fluid aufnehmendes Verhalten zeigt. Die weiche, porige Einlage 8 kann als Schwamm, Membran, Vlies oder sonstiges textiles Material aus Natur- und/oder Kunststofffasern ausgebildet sein. Als Schwamm besonders geeignet ist beispielsweise ein PU-Schaum, der eine gute Saugfähigkeit besitzt.

Wesentlich ist, dass die Einlage 8 ein Fluid aufnehmendes Verhalten zeigt, wenn es beim Andrücken der Schutzkappe 7 gegen das Austragabschnittsende 5 gedrückt wird. Erstreckt sich die Einlage 8 von der kopfseitigen Schutzkappenwand 12 mit einer Axiallänge in den Innenraum der Schutzkappe 7, wird die Einlage 8 beim Aufsetzen und Andrücken der Schutzkappe 7 auf eine Kopfwand 13 des Austragabschnittsendes 5 gedrückt. In der Kopfwand 13 ist die Austragsöffnung 4 angeordnet. Die Einlage 8 besitzt vorzugsweise eine radiale Erstreckung, die größer ist als der Durchmesser der Austragsöffnung 4, wie Fig. 4 zeigt. Die Einlage 8 kommt somit bei ihrem Aufdrücken in Kontakt mit einem Bereich, der von der Kopfwand 13 um den Randbereich der Austragsöffnung 4 gebildet wird. Medienreste, die bei einem Austraghub oder Sprühvorgang im Bereich um die Austragsöffnung 4 verbleiben, können so von der Einlage 8 erfasst und aufgenommen werden. Dieses Andrücken der Einlage 8 zur Abnahme von Medienresten vom Austragabschnittsende 5 in der Pfeilrichtung X (vgl. Fig. 5) ist erfindungsgemäß kombiniert mit einer Bewegungskomponente in Pfeilrichtung Y, um ein quasi reibendes Darüberwischen zu erzeugen, wodurch die Fluidaufnahme verbessert ist.

Die hierzu vorgesehene Torsionsfeder 11 ist beispielsweise eine Drehstabfeder, Schraubenfeder oder Gummifeder. Die Torsionsfeder 11 ist stabförmig mit einer vorzugsweise axial gerichteten federnden Länge im Innenraum der Schutzkappe 7 angeordnet. Die Einlage 8 ist vorzugsweise von der Torsionsfeder 11 umgeben. Die Einlage 8 und die Torsionsfeder 11 werden dann gemeinsam beim Aufdrücken der Schutzkappe 7 beansprucht. Die Torsionsfeder 11 ist also ein elastischer Stab, dessen eines Ende an der Schutzkappe 7 eingespannt ist und an dessen freiem Ende 10 bei Belastung ein Drehmoment eingeleitet wird. Die Folge ist, dass sich das freie Ende 10 gegenüber dem eingespannten Ende und damit die lose Schutzkappe 7 unter einer angewendeten manuellen Andruckkraft in Pfeilrichtung X gegenüber dem Austragabschnitt 3 in Pfeilrichtung Y zumindest um einen kleinen Winkel verdreht.

Zur Unterbringung der Einlage 8 und der Torsionsfeder 11 im Innenraum der Schutzkappe 7 besitzt die Schutzkappe 7 vorzugsweise eine Überlänge gegenüber dem mindestens zu überdeckenden Austragabschnittsende 5. Bei der im Nichtgebrauchszustand vorzugsweise fest aufgesetzten Schutzkappe 7 sind die Einlage 8 und die Torsionsfeder 11 dann entlastet oder zumindest teilweise entlastet.

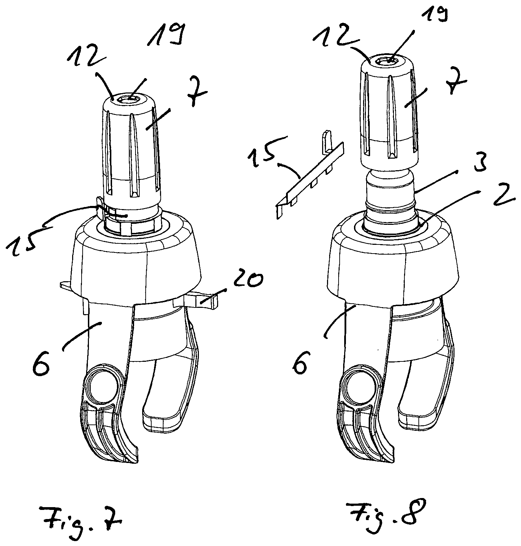

Wie die Fig. 3 bis Fig. 6 weiterhin zeigen, ist die Schutzkappe 7 vorzugsweise über eine Schnappverbindung 14 an dem Austragabschnitt 3 abnehmbar befestigbar. Fig. 4 zeigt die Schnappelemente dieser Schnappverbindung 14 vor einem Erstgebrauch in einem noch nicht verrasteten Zustand. Vor dem Erstgebrauch ist ein Abreißring 15 zwischen der Schutzkappe 7 und der Betätigungseinrichtung 6 vorgesehen, um einen Erstgebrauch anzuzeigen. Die Fig. 7 und Fig. 8 zeigen das Entfernen des Abreißringes 15 für den Erstgebrauch. Erst nach Abtrennung des Abreißringes 15 kann die Schutzkappe 7 abgenommen und das Pumpenteil 2 zum Austrag von Medium betätigt werden.

Wie Fig. 4 zeigt, umfasst die Schnappverbindung 14 als eine segmentierte Ring-Schnapp-Verbindung an dem Austragabschnitt 3 einen endfernen Außenwulst 16, der als ein Formteil angeordnet ist. An dem Außenwulst 16 kann die Schutzkappe 7 deckelartig mit segmentierten endnahen Innenringschnappelementen 17 lösbar rasten. Vor dem Erstgebrauch, den Fig. 4 zeigt, ist noch keine Fügeverbindung zwischen dem Außenwulst 16 und den Innenringschnappelementen 17 gegeben, da die Innenringschnappelemente 17 oberhalb des Außenwulstes 16 liegen. Vor einem Erstgebrauch entfällt die Notwendigkeit eines Abwischens von Medienresten.

Wie Fig. 6 gegenüber Fig. 5 zeigt, besitzt die Torsionsfeder 11 vorzugsweise eine federnde Länge, die über den Übergang der Rastelemente der Schnappverbindung 14, der durch den Außenwulst 16 der Schnappverbindung 14 bestimmt wird, hinausgeht. Die federnde Länge der Torsionsfeder 11 wird durch einen Anschlag 18 bestimmt, der einen Überschwingungsweg der Schutzkappe 7 gegenüber einem Schnappelement, hier der Außenwulst 16, an dem Austragabschnitt 3 begrenzt. Bei Anwendung einer manuellen Kraft zum Drücken der Schutzkappe 7 in die Schnappverbindung 14 wird ein quasi Zwangsnachlauf der Schutzkappe 7 bis zum Anschlag 18 erzeugt. Die Torsionsfeder 11 wirkt dabei auch noch als Dämpfungsglied, so dass die erzeugte Verdrehbewegung des Federelements einen elastischen Eindruck hinterlässt. Der Anschlag 18 kann getrennt vom Austragabschnitt 3 ausgebildet sein, da er diesem nur zugeordnet zu sein braucht. Die Fig. 5 zeigt die Schutzkappe 7 beim Anschlag gegen den Anschlag 18. Beim Entlasten der Schutzkappe 7 bewegt sich diese nach oben, bis die Innenringschnappelemente 17 den Außenwulst 16 als lösbare Fügeposition hintergreifen (nicht dargestellt). Die Torsionsfeder 11 hält die Fügeposition der Schnappverbindung 14 vorzugsweise unter einer Federvorspannung.

Die Schutzkappe 7 kann eine oder mehrere Öffnungen 19 im Bereich der Umhüllung der Einlage 8 zur Ausbildung eines luftdurchlässigen Verschlusses aufweisen. Hier ist/sind die Öffnungen 19 beispielsweise in der Schutzkappenwand 12 angeordnet. Die Schutzkappe 7 ist dadurch besser ventilierbar für ein Abtrocknen der weichen, porigen Einlage 8 während des Nichtgebrauchs des Spenders.

Als Material für die Torsionsfeder 11 ist beispielsweise Polypropylen (PP), Polyoxymethylen (POM) oder Polybutylenterephthalat (PBT) vorgesehen. Die Abdeckungslänge der Schutzkappe 7 gegenüber dem Austragabschnitt 3 ist anwendungsabhängig wählbar. Bei einem Nasenadapter ist beispielsweise eine längere Schutzkappe zur Abdeckung vorgesehen als bei einem Tropfadapter. In bekannter Weise kann ein einsetzbarer Betätigungsstopper 20 für das Pumpenteil 2 vorgesehen sein.

Die weiche, porige Einlage 8 kann auch ein stäbchenförmiger Träger sein, der für Filterzwecke oder Analysezwecke, wie eine DNA-Bestimmung, als Sammler auswechselbar sein kann.

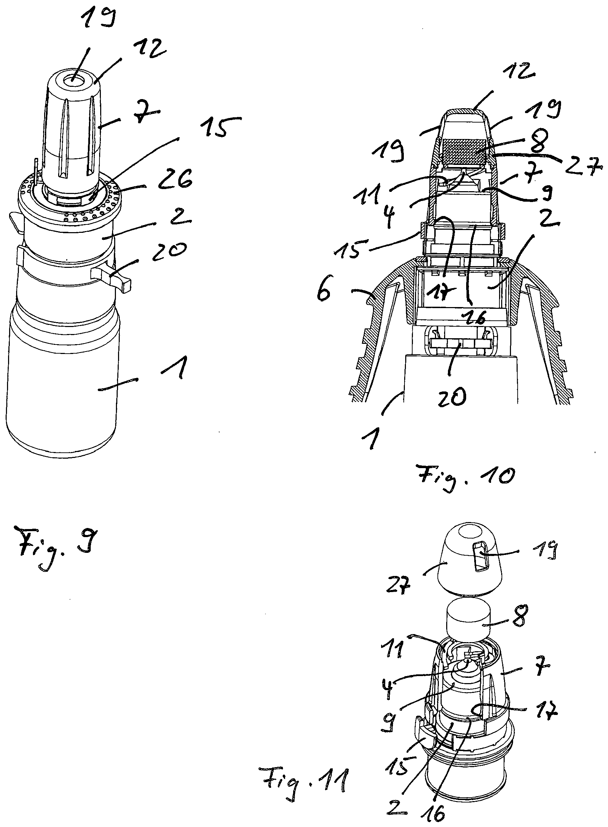

Fig. 9 zeigt ein zweites Ausführungsbeispiel eines manuell betätigbaren Spenders, das sich von dem zuvor beschriebenen Ausführungsbeispiel dadurch unterscheidet, dass eine andere Betätigungseinrichtung 26 für das Pumpenteil 2 vorgesehen ist. Die Betätigungseinrichtung 26 ist hier in bekannter Weise als ein Handle-Teller ausgebildet. Im Übrigen gelten die vorstehenden Ausführungen entsprechend. Die Ausbildung der Schutzkappe 7 ist unabhängig von der Art und Richtung der angreifenden Betätigungseinrichtung.

Fig. 10 und Fig. 11 zeigen ein drittes Ausführungsbeispiel eines manuell betätigbaren Spenders, das sich von dem zuvor beschriebenen ersten Ausführungsbeispiel dadurch unterscheidet, dass die Schutzkappe 7 einen lösbar anbringbaren Kopfabschnitt 27 aufweist. Dies ist besonders dann vorteilhaft, wenn die Einlage 8 häufiger ausgetauscht oder als Sammler für Analysezwecke Verwendung findet. Dann kann es sein, dass die Einlage 8 bereits nach einem oder wenigen Pumpenhüben ausgetauscht wird. Die Torsionsfeder 11 ist dann vorzugsweise kurz ausgebildet, beispielsweise 5 bis 10 mm und/oder nur zwei bis drei Windungen, mit der Folge, dass dann nur eine geringe Schwenkbewegung um die Drehachse bei einer Belastung der Torsionsfeder 11 auftritt. Dies kann mindestens teilweise kompensiert werden durch eine voluminöse Einlage 8, die bei Verwendung von elastischem Material aufgrund ihres Stauchverhaltens eine Axialbewegung mit Schwenkbewegungen kombinieren kann. Der dadurch ausgelöste Saug- und Wischeffekt ist zur Abnahme von Medium von der Austragöffnung 4 in die Einlage 8 hinreichend gut geeignet. Die Einlage 8 kann dazu eine geformte Einlage sein, die beispielsweise zapfenförmig oder zapfenabschnittsförmig ist.

## Patentansprüche

1. Manuell betätigbarer Spender für Medien mit einem Vorratsbehälter (1), einem auf dem Vorratsbehälter (1) aufgesetzten Pumpenteil (2), das einen stutzenförmigen Austragabschnitt (3) mit einem eine Austragöffnung (4) aufweisenden Austragabschnittsende (5) umfasst, einer dem Pumpenteil (2) zugeordneten Betätigungseinrichtung (6, 26) und einer mindestens das Austragabschnittsende (5) abdeckenden Schutzkappe (7), **dadurch gekennzeichnet, dass** in die Schutzkappe (7) eine weiche, porige Einlage (8) eingelegt ist, die beim Aufsetzen der Schutzkappe (7) von oben an das Austragabschnittsende (5) drückbar ist, und der Austragabschnitt (3) eine Aufstandsfläche (9) für ein freies Ende (10) einer in der Schutzkappe (7) eingespannten Torsionsfeder (11) bildet, deren Belastung ein Drehmoment einleitet, das die Schutzkappe (7) gegenüber dem Austragabschnitt (3) verdreht.

2. Manuell betätigbarer Spender nach Anspruch 1, **dadurch gekennzeichnet, dass** die Torsionsfeder (11) eine Drehstabfeder, Schraubenfeder oder Gummifeder ist.

3. Manuell betätigbarer Spender nach Anspruch 1 oder 2, **dadurch gekennzeichnet, dass** die weiche, porige Einlage (8) als Schwamm, Membran, Vlies oder sonstiges textiles Material aus Natur- und/oder Kunststofffasern ausgebildet ist.

4. Manuell betätigbarer Spender nach einem der Ansprüche 1 bis 3, **dadurch gekennzeichnet, dass** die weiche, porige Einlage (8) kissenartig ausgebildet ist.

5. Manuell betätigbarer Spender nach einem der Ansprüche 1 bis 4, **dadurch gekennzeichnet, dass** die weiche, porige Einlage (8) von der Torsionsfeder (11) umgeben ist.

6. Manuell betätigbarer Spender nach einem der Ansprüche 1 bis 5, **dadurch gekennzeichnet, dass** die Schutzkappe (7) über eine Schnappverbindung (14) an dem Austragabschnitt (3) abnehmbar befestigbar ist.

7. Manuell betätigbarer Spender nach Anspruch 6, **dadurch gekennzeichnet, dass** die Schnappverbindung (14) als segmentierte Ring-Schnapp-Verbindung ausgebildet ist.

8. Manuell betätigbarer Spender nach Anspruch 6 oder 7, **dadurch gekennzeichnet, dass** die Torsionsfeder (11) die Fügeposition der Schnappverbindung (14) unter einer Federvorspannung hält.

9. Manuell betätigbarer Spender nach einem der Ansprüche 6 bis 8, **dadurch gekennzeichnet, dass** die Torsionsfeder (11) eine federnde Länge besitzt, die über den Übergang der Rastelemente der Schnappverbindung (14) hinausgeht.

10. Manuell betätigbarer Spender nach Anspruch 9, **dadurch gekennzeichnet, dass** die federnde Länge durch einen Anschlag (18) begrenzt ist, der einen Überschwingungsweg der Schutzkappe (7) gegenüber einem Schnappelement an dem Austragabschnitt (3) begrenzt.

11. Manuell betätigbarer Spender nach einem der Ansprüche 6 bis 10, **dadurch gekennzeichnet, dass** an dem Austragabschnitt (3) ein endferner Außenwulst (16) als Formteil angeordnet ist, an dem die Schutzkappe (7) deckelartig mit segmentierten endnahen Innenringschnappelementen (17) lösbar rastet.

12. Manuell betätigbarer Spender nach einem der Ansprüche 1 bis 11, **dadurch gekennzeichnet, dass** die Schutzkappe (7) Öffnungen (19) im Bereich der Umhüllung der weichen, porigen Einlage (8) zur Ausbildung eines luftdurchlässigen Verschlusses aufweist.

## Claims

1. Manually operated dispenser for media, with a reservoir (1), with a pump part (2) which is mounted on the reservoir (1) and comprises a nozzle-shaped discharge portion (3) with a discharge portion end (5) having a discharge opening (4), with an actuation mechanism (6, 26) assigned to the pump part (2), and with a protective cap (7) covering at least the discharge portion end (5), **characterized in that** a soft porous insert (8) is inserted into the protective cap (7) and can be pressed when the protective cap (7) is fitted from above onto the discharge portion end (5), and the discharge portion (3) forms a stand surface (9) for a free end (10) of a torsion spring (11), which is clamped in the protective cap (7) and whose loading introduces a torque that turns the protective cap (7) relative to the discharge portion (3).

2. Manually operated dispenser according to Claim 1, **characterized in that** the torsion spring (11) is a torsion bar spring, helical spring or rubber spring.

3. Manually operated dispenser according to Claim 1 or 2, **characterized in that** the soft porous insert (8) is designed as sponge, membrane, nonwoven or other textile material from natural and/or synthetic fibres.

4. Manually operated dispenser according to one of Claims 1 to 3, **characterized in that** the soft porous insert (8) is designed like a cushion.

5. Manually operated dispenser according to one of Claims 1 to 4, **characterized in that** the soft porous insert (8) is surrounded by the torsion spring (11).

6. Manually operated dispenser according to one of Claims 1 to 5, **characterized in that** the protective cap (7) can be secured removably on the discharge portion (3) by a snap-fit connection (14).

7. Manually operated dispenser according to Claim 6, **characterized in that** the snap-fit connection (14) is designed as a segmented ring snap-fit connection.

8. Manually operated dispenser according to Claim 6 or 7, **characterized in that** the torsion spring (11) holds the joining position of the snap-fit connection (14) under spring pretensioning.

9. Manually operated dispenser according to one of Claims 6 to 8, **characterized in that** the torsion spring (11) has a resilient length that goes beyond the transition of the locking elements of the snap-fit connection (14).

10. Manually operated dispenser according to Claim 9, **characterized in that** the resilient length is limited by a stop (18), which limits an overshoot path of the protective cap (7) relative to a snap-fit element on the discharge portion (3).

11. Manually operated dispenser according to one of Claims 6 to 10, **characterized in that** an outer bead (16) far from the end is arranged as shaped part on the discharge portion (3), on which outer bead (16) the protective cap (7) locks releasably like a lid via segmented inner ring snap-fit elements (17) near the end.

12. Manually operated dispenser according to one of Claims 1 to 11, **characterized in that** the protective cap (7) has openings (19), in the area where the soft porous insert (8) is enclosed, in order to form an air-permeable closure.

## Revendications

1. Distributeur actionnable manuellement pour des milieux comprenant un réservoir de stockage (1), une partie de pompe (2) montée sur le réservoir de stockage (1) et qui comprend une section de distribution (3) en forme de tubulure présentant une extrémité (5) de section de distribution munie d'une ouverture de distribution (4), un dispositif d'actionnement (6, 26) associé à la partie de pompe (2) et un capuchon de protection (7) couvrant au moins l'extrémité (5) de la section de distribution, **caractérisé en ce qu'**un insert (8) souple et poreux est disposé dans le capuchon de protection (7) qui lors de la mise en place du capuchon de protection (7) peut être pressé depuis le dessus sur l'extrémité (5) de section de distribution et la section de distribution (3) forme une surface de support (9) pour une extrémité libre (10) d'un ressort de torsion (11) serré dans le capuchon de protection (7), et dont la charge imprime un moment de torsion qui fait tourner le capuchon de protection (7) par rapport à la section de distribution (3).

2. Distributeur actionnable manuellement selon la revendication 1, **caractérisé en ce que** le ressort de torsion (11) est une barre de torsion, un ressort hélicoïdal, ou un ressort caoutchouc.

3. Distributeur actionnable manuellement selon la revendication 1 ou 2, **caractérisé en ce que** l'insert (8) souple et poreux est réalisé sous la forme d'une éponge, d'une membrane, d'une matière non tissée ou d'un autre matériau textile en fibres naturelles et/ou synthétiques.

4. Distributeur actionnable manuellement selon l'une des revendications 1 à 3, **caractérisé en ce que** l'insert (8) souple et poreux est conformé en forme de coussin.

5. Distributeur actionnable manuellement selon l'une des revendication 1 à 4, **caractérisé en ce que** l'insert (8) souple et poreux est entouré du ressort de torsion (11).

6. Distributeur actionnable manuellement selon l'une des revendications 1 à 5, **caractérisé en ce que** le capuchon de protection (7) peut être fixé de manière amovible sur la section de distribution (3) au moyen d'une connexion par enclenchement élastique (14).

7. Distributeur actionnable manuellement selon la revendication 6, **caractérisé en ce que** la connexion par enclenchement élastique (14) est conformée sous la forme d'une connexion par enclenchement élastique en anneau segmentée.

8. Distributeur actionnable manuellement selon la revendication 6 ou 7, **caractérisé en ce que** le ressort de torsion (11) maintien la position de connexion de la connexion par enclenchement élastique (14) sous une précharge élastique.

9. Distributeur actionnable manuellement selon l'une des revendications 6 à 8, **caractérisé en ce que** le ressort de torsion (11) présente une longueur élastique qui va au-delà de la transition des éléments de verrouillage de la connexion par enclenchement élastique (14).

10. Distributeur actionnable manuellement selon la revendication 9, **caractérisé en ce que** la longueur élastique est limitée par une butée (18) qui limite une course de dépassement du capuchon de protection (7) par rapport à un élément d'enclenchement élastique sur la section de distribution (3).

11. Distributeur actionnable manuellement selon l'une des revendications 6 à 10, **caractérisé en ce qu'**un renflement extérieur (16) à distance de l'extrémité est ménagé en tant que pièce moulée sur la section de distribution (3) sur lequel le capuchon de protection (7) se verrouille de manière amovible comme un couvercle via les éléments d'enclenchement élastique en anneau intérieure segmentés (17) proches de l'extrémité.

12. Distributeur actionnable manuellement selon l'une des revendications 1 à 11, **caractérisé en ce que** le capuchon de protection (7) présente des ouvertures (19) dans la région de l'enveloppe de l'insert (8) souple et poreux afin de former une fermeture perméable à l'air.
